# EUROPEAN PATENT APPLICATION

(11) **EP 4 652 936 A1**
(43) Date of publication of application: **26.11.2025**
(21) Application number: 25178329.6
(22) Date of filing: 22.05.2025
(51) Int. Cl.: A61B 7/00, A61B 7/04

(54) **WEARABLE PHYSIOLOGICAL SIGNAL SENSING SYSTEM**

(30) Priority: 23.05.2024 TW 113119137; 25.12.2024 TW 113150770
(71) Applicant: Decentralized Biotechnology Intelligence Co., Ltd., Taipei 106021 (TW)
(72) Inventor: CHOU, Yao-Sheng, 106021 Taipei CIty (TW); JIANG, Lin-Yi, 106021 Taipei City (TW); LIN, Hsiao-Yi, 106021 Taipei City (TW); CHOU, Yen-Han, 106021 Taipei city (TW)
(74) Representative: Lermer, Christoph

(57) **Abstract**

The present invention discloses a wearable sensing system for detecting physiological signals, which includes a system circuit board with upper and lower surfaces, a stethoscope is disposed on the lower surface for sensing the user's heart sound signal, and a plurality of electrocardiographic electrodes is disposed on the lower surface and adjacent to the stethoscope used to sense the user's ECG signal, and an oximeter is disposed on the upper surface to sense the user's blood oxygen concentration and pulse wave signal. The system circuit board is electrically connected to the stethoscope, the plurality of ECG electrodes and the oximeter. The system circuit board obtains the user's pulse transit time (PPT) by comparing the ECG signal with the pulse wave signal or by comparing the heart sound signal with the pulse wave signal. The PPT is used to calculate the user's continuous blood pressure.

## Description

### TECHNICAL FIELD

The present invention relates to a medical equipment, and more particularly, a wearable physiological signal sensing system.

### BACKGROUND

As people pay more attention to their own health conditions, physiological monitoring systems are becoming more consummate. For patients with chronic diseases, long-term and accurate physiological testing may effectively reduce the risk of disease, and provide effective data reference for treatment. Traditional physiological detection equipment is employed by medical and health care instruments to measure heart rate, respiratory rate, blood pressure, blood oxygen level and body temperature, it provides a basis for measuring the health status of the blood circulation and respiratory systems. It is also widely used in home health care. It has broad application prospects in remote medical care and clinical medicine as well.

The medical testing instrument towards the trend of portability and networking. The traditional physiological testing instruments have only single function and suffer large size. Therefore, they cannot meet the increasing demands for long-term and real-time testing. As sensor technology becomes more integrated and intelligent, it is possible to integrate multiple sensors into one single device for medical testing while keeping costs low.

Heart failure is a worldwide public health problem and it causes huge burden on overall medical costs. In recent years, it becomes popular to monitor physical and mental states in daily life by long-term recording and analyzing physiological information from several hours to months. Heart sound detection equipment, such as a stethoscope, is an instrument that diagnoses organ activity by detecting sounds. An electronic stethoscope collects the sounds of the organ activity such as the heart and lungs by placing the stethoscope head on the corresponding part of the organism being tested, converting these sounds into electrical signals, which are then amplified and output from a speaker, so that doctors can determine the cause or lesion based on the corresponding sound signals and make a correct diagnosis.

With the aging of the population, the health care and monitoring products are popular nowadays. To detect symptoms in advance, especially for cardiac diseases with a high sudden death rate, the wearable physiological signal sensing systems may provide real-time, effective detection and record the abnormal heartbeat signals. By placing detection devices on certain areas of the chest wall, heart sounds can be heard. Certain abnormal heart movements can cause murmurs or other abnormal heart sounds. Therefore, listening to heart sounds or recording phonocardiogram (PCG) can effectively make up for the shortcomings of cardiac auscultation. It may also provide physiological signals such as blood oxygen level and blood pressure. Doctors may use these real-time recorded physiological signals to analyze, thereby providing health suggestions and care solutions. By monitoring this physiological information in daily life, it can be effectively used to improve health or early detection of diseases.

Therefore, what is required is to provide an advanced wearable physiological signal detection system for home/ambulatory care, health management and autonomous health warning.

### SUMMARY OF THE INVENTION

According to the purpose of the present invention, the present invention discloses a wearable physiological signal sensing system which comprises a system circuit board having the upper surface and the lower surface. A stethoscope is disposed on the lower surface for sensing the user's heart sound signal, and ECG electrodes are arranged on the lower surface and adjacent to the stethoscope for sensing the ECG signal of the user. An oximeter is arranged on the upper surface for sensing the blood oxygen level and pulse wave signal of the user. The system circuit board is electrically connected to the stethoscope, the plurality of ECG electrodes and the blood oximeter. The system circuit board is instructed to compare the ECG signal with the pulse wave signal or compare the heart sound signal with the pulse wave signal to obtain the user's pulse wave transit time which is used to calculate the user's continuous blood pressure.

In one embodiment, the continuous blood pressure of the user is estimated by an artificial intelligence (AI) algorithm. A multivariate linear model is established by the artificial intelligence algorithm using the time domain characteristics of the pulse wave signal, the pulse wave transit time and the user's height parameters.

In one embodiment, the stethoscope includes a diaphragm, a piezoelectric sensor, and a sound isolation ring. The diaphragm is disposed on the lower surface of the circuit board; the soundproof ring is disposed on the lower surface and surrounds the diaphragm. The soundproof ring is packaged with the circuit board to form a resonance cavity. The piezoelectric sensor is arranged on the soundproof ring side that is not in contact with the circuit board. In one case, the piezoelectric sensor is attached to the user's skin.

In one embodiment, the blood oximeter includes an infrared light source, a red-light source, and a photoreceptor for sensing the user's fingertip pulse wave. The circuit board at least includes a signal preprocessing circuit for filtering, amplifying and converting the heart sound, electrocardiogram and pulse wave signals. A microprocessor is used for receiving the digitized heart sound, electrocardiogram and pulse wave signals, and followed by processing these signals to obtain pre-processed digitized heart sound, electrocardiogram and pulse wave signals.

In one embodiment, the wearable physiological signal sensing system is attached to the chest of the user to continuously monitor the heart sound signals and electrocardiogram signals. The blood oxygen level and pulse wave signal are obtained by the user while pressing the oximeter.

In one embodiment, the wearable physiological signal sensing system is connected to an external mobile device to transmit the physiological signals. The external mobile device is connected to a cloud server or an edge computing device to process and analyze the physiological signals. The signal includes one or any combination of electrocardiogram signal, pulse wave signal, heart sound signal and blood oxygen level. In another embodiment, the wearable physiological signal sensing system includes an e-SIM disposed on the system circuit board. In one embodiment, the wearable physiological signal sensing system communicates with the external mobile device, the cloud server or the edge computing device by the e-SIM to transmit the physiological signal.

In one embodiment, the system circuit board performs the following steps to measure the blood pressure, the steps include sensing a user's heart sound signal, electrocardiogram signal and the blood oxygen saturation level; comparing the pulse wave with the electrocardiogram signal, or comparing the heart sound signal with the pulse wave signal to obtain the pulse wave transit time, followed by obtaining the user's blood pressure by the AI algorithm.

In one embodiment, the wearable physiological signal sensing system includes the system circuit board having the upper surface and the lower surface; a stethoscope is disposed on the lower surface and electrically connected to the system circuit board for sensing the user's physiological signal. At least one patch is arranged on the lower surface for adhering to the user's skin. Plurality of ECG electrodes are disposed on the lower surface for sensing the user's ECG signals. The oximeter is arranged on the upper surface and is used to sense the blood oxygen level and pulse wave signal of the user. In one embodiment, the method includes step of comparing the electrocardiogram signal with the pulse wave signal, or comparing the heart sound signal with the pulse wave signal to obtain the pulse wave transit time of the user, to calculate the continuous blood pressure of the user; wherein the blood pressure is predicted based on the continuous blood pressure by the artificial intelligence algorithm. A multivariate linear model is established by the AI algorithm using the time domain characteristics of the pulse wave signal, the pulse wave transit time and the user's height.

### BRIEF DESCRIPTION OF THE DRAWINGS

FIG. 1 illustrates a system according to one embodiment of the present invention.
FIG. 2(a) shows a side view of the piezoelectric sensor structure according to the present invention.
FIG. 2(b) shows a piezoelectric patch structure according to the present invention.
FIG. 3(a) - FIG. 3(c) respectively show different design embodiments of the piezoelectric patch structure.
FIG. 4(a) - FIG. 4(c) show the method of attaching the piezoelectric patch structure for testing.
FIG. 5(a) - FIG. 5(c) show an embodiment of a pressure sensor configuration according to the present invention.
FIG. 6(a) shows a stethoscope according to an embodiment of the present invention.
FIG. 6(b) shows another stethoscope embodiment of the present invention.
FIG. 7 shows a functional block diagram of the stethoscope according to the present invention.
FIG. 8 shows a flow chart of the heart sound detection system according to an embodiment of the present invention.
FIG. 9 shows a schematic diagram of an embodiment of the charging state according to the present invention.
FIG. 10 is a schematic diagram showing an embodiment of a charging state according to the present invention.
FIG. 11 illustrates a method for obtaining pulse wave transit time according to one embodiment of the present invention.
FIG. 12 illustrates a method of measuring blood pressure according to one embodiment of the present invention.
FIG. 13(a) illustrates a wearable physiological signal sensing system according to one embodiment of the present invention.
FIG. 13(b) illustrates a wearable physiological signal sensing system according to an alternative embodiment of the present invention.
FIG. 13(c) illustrates a wearable physiological signal sensing system according to another embodiment of the present invention.
FIG. 13(d) illustrates a wearable physiological signal sensing system according to alternative embodiment of the present invention.
FIG. 14 illustrates a function diagram of a wearable physiological signal sensing system according to one embodiment of the present invention.
FIG. 15(a) illustrates a system structure according to one embodiment of the present invention.
FIG. 15(b) illustrates an alternative system structure according to one embodiment of the present invention.
FIG. 16 illustrates a process system according to one embodiment of the present invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

The present invention discloses a wearable physiological sound detection system, which mainly utilizes a heart sound detection device worn on the human body. The wearable physiological sound collection device of the present invention integrates with a sound sensing device and a wireless device, and it can connect to the Internet of Things. The collected physiological data are processed and sent by a portable electronic computing device (for example, mobile device) to a cloud server via a cloud network. The present invention also discloses a wearable physiological sensing system for real-time monitoring key physiological signals including one or any combination of heart, lung sounds, electrocardiogram, blood oxygen saturation level and blood pressure.

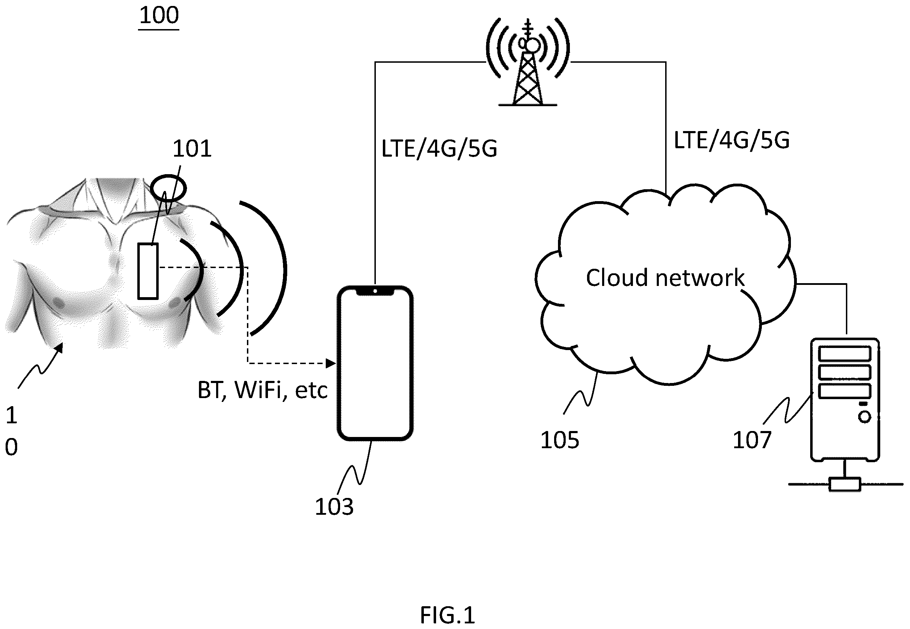

FIG. 1 shows the wearable physiological sound detection system 100, which includes a physiological sound collecting device 101 attached to a user 10 in the form of a monitoring patch. The physiological sound collecting device 101 is communicatively connected to a mobile device (e.g., an external computing electronic device such as a smart phone or a tablet computer) 103. The physiological sound data collected by the wearable physiological sound collecting device 101 can be uploaded to the cloud server 107 by the mobile device 103 via the cloud network 105 through wireless transmission (such as WiFi, LTE/4G, 5G, 6G and their update version). The data will be stored in the cloud data database. An application (APP) is installed in the mobile device 103, wherein the application includes instructions for receiving and sending data among the wearable physiological sound collecting device 101, the mobile device 103 and the cloud server 105. The application can be operated based on Android, Windows or iOS operating system platforms, and uploads the collected data/signals, such as heart sound signals and their waveforms, to the cloud server 105 for storage, and the data is processed by feature extraction algorithms to generate an evaluation report for providing medical advice based on the data.

The physiological sound collecting device (stethoscope) 101 is attached to the chest of the user 10 in the form of a patch. It senses the human body's sound signals through its built-in acoustic device. The acoustic device includes piezoelectric sensors and microphones. The piezoelectric sensor is composed of a piezoelectric material layer (for example, polyvinylidene fluoride (PVDF) polymer piezoelectric film, lead zirconate titanate (PZT) and other materials), its upper and lower surfaces are plated with conductive metals (for example, aluminum (Al), copper (Cu), etc.). Leads are out from each of the upper and lower metal layers and connected to the circuit board, the lead is used to measure the voltage signal generated by vibration. The component of the microphone includes a capacitive sensor having an ultra-thin material as a diaphragm (for example, 30 µm thick glass), the diaphragm is plated with conductive material, and the diaphragm is sealed with a circuit board by means of a sealant, thereby forming a resonance chamber. The purpose is to use the sound of the heartbeat to vibrate the diaphragm, causing a change in capacitance between the diaphragm and the circuit board. The stethoscope captures this change to record the heartbeat.

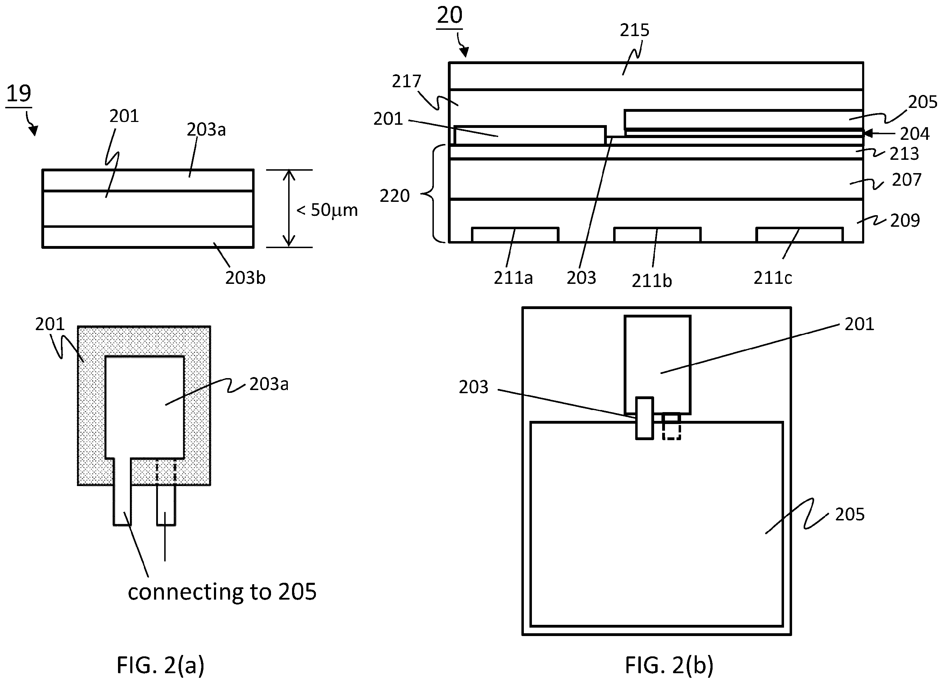

Referring to FIG. 2(a), a side view of the piezoelectric sensor structure 19 of the present invention is shown. The upper part of the figure shows that the piezoelectric sensor which is composed of a piezoelectric material layer (for example, polyvinylidene fluoride (PVDF) polymer piezoelectric film, lead zirconate titanate (PZT) and other materials) 201, and the upper and lower surfaces are plated with conductive metals (for example, aluminum (Al), copper (Cu), etc.) to act as an upper electrode 203a and a lower metal electrode 203b, each of them has a wire connected to the circuit board 205, the upper electrode 203a and the lower metal electrode 203b are used to measure the voltage signal generated by vibration. In one embodiment, the thickness of the piezoelectric sensor is less than 50 µm.

FIG. 2(b) shows the structure of the piezoelectric patch 20 of the present invention. From the side view of the piezoelectric patch, it includes a substrate 207, a layer of anti-allergic gel 209 is coated on the bottom of the substrate 207 to contact the skin, a plurality of bottom electrodes (211a, 211b, 211c) are arranged under the anti-allergic gel 209 to expose parts of the outer surface of the gel layer 209. The bottom electrodes (211a, 211b, 211c) are used to determine whether the piezoelectric patch 20 is attached properly or not. An insulating layer 213 is arranged on the substrate 207 as a planarization layer. The piezoelectric material layer 201 is attached to the insulating layer 213. The metal lead 203 extends to connect with the circuit board 205 located adjacent to the lead 203 by an electrical connection 204, such as conductive glue, a snap-on connector, etc., the cover plate 215 is attached by a packaging glue 217 to serve as a protective layer. The insulating layer 213, the substrate 207, and the gel layer 209 are stacked from bottom to top to form a base 220. The lower part of the figure 2(b) is a front view of the piezoelectric patch structure 20 of the present invention.

In one embodiment, the material of the substrate 207 can be textile, glass or plastic such as polyimide (PI), polyethylene terephthalate (PET). The packaging glue 217 is ethylene vinyl acetate polymer (EVA). The cover plate 215 is made of glass or plastic such as polyimide (PI) or polyethylene terephthalate (PET), or it may be made of textile.

In one embodiment, the thickness of the piezoelectric patch structure 20 is less than 2000 µm, the thickness of the gel layer 209 is less than 700 µm, the thickness of the substrate 207 is less than 300 µm, the thickness of the insulating layer 213 is less than 50 µm, the thickness of the piezoelectric material layer 201 is less than 50 µm, the thickness of the circuit board 205 is less than 200 µm; and the thickness of the packaging glue 217 is less than 300 µm. In one embodiment, the piezoelectric sensor may be replaced by an acceleration sensor, a gyroscope or other sensors.

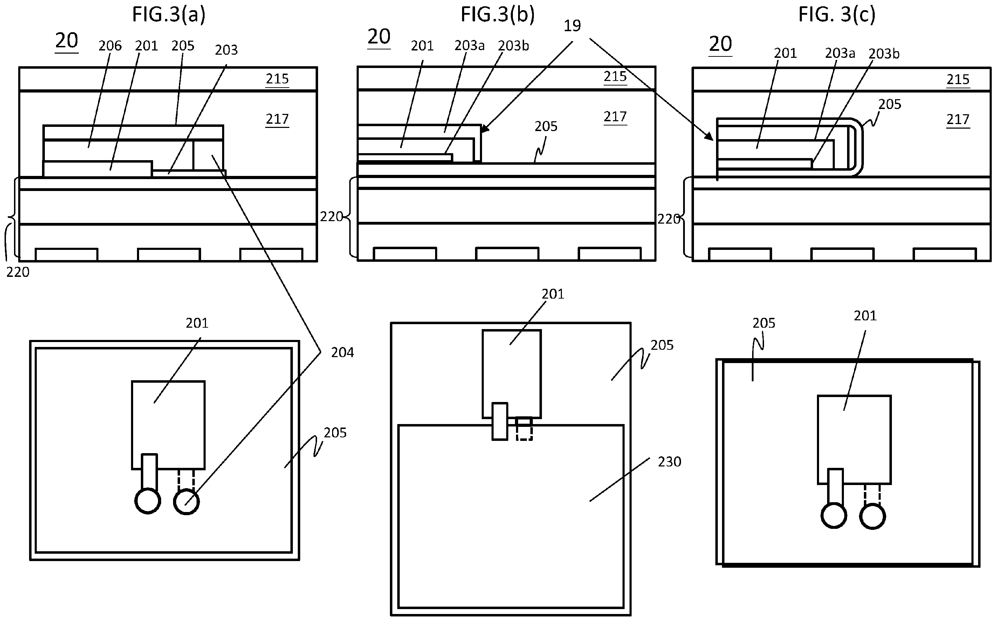

Figures 3(a)-(c) respectively show different designs of the piezoelectric patch structure 20. In Figure 3(a), the circuit board 205 is set above the piezoelectric material 201. The piezoelectric material layer 201 is attached to the insulating layer 213. The circuit board 205 is attached to the piezoelectric material 201 through an adhesive (paste) 206, and the electrical connection 204 between the circuit board 205 and the metal lead 203 plated on the piezoelectric material 201 is formed by conductive glue or a snap-on connector. FIG. 3(b) shows that the piezoelectric material 201 is directly attached to the circuit board 205, and the metal lead 203 of the piezoelectric sensor structure 19 is electrically connected to a system board 230. FIG. 3(c) shows that the piezoelectric material 201 is directly attached to the circuit board 205, and the circuit board 205 is a flexible circuit board which is bent directly onto the piezoelectric sensor structure 19.

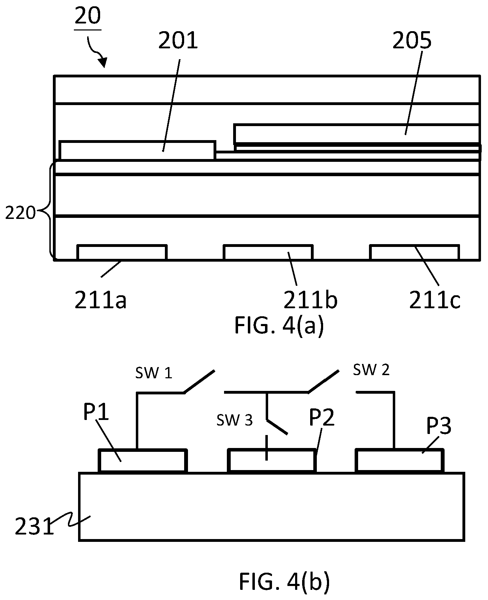

Figure 4(a) shows a method for attaching and testing the piezoelectric patch 20. In one case, the stacking structure is shown in Figure 2(b). Please refer to the previous description for structural details. The structure includes a plurality of bottom electrodes (211a, 211b, 211c) arranged under the gel layer 209. The multiple bottom electrodes (211a, 211b, 211c) are referred as the first electrode P1, the second electrode P2 and the third electrode P3. As shown in Figure 4(b), a switch circuit is configurated between the multiple bottom electrodes for attaching the piezoelectric patch 20 to the skin resistance 231 of the human skin to perform an adhesion test. The switch circuit is configured to connect the first electrode P1 and the third electrode P3 in a loop by the first switch SW1 (switch 1) and the second switch SW2 (switch 2) in series, to connect the first electrode P1 and the second electrode P2 in a loop by the first switch SW1 (switch 1) and the third switch SW3 (switch 3) in series, and to connect the third electrode P3 and the second electrode P2 in a loop by the second switch SW2 (switch 2) and the third switch SW3 (switch 3) in series.

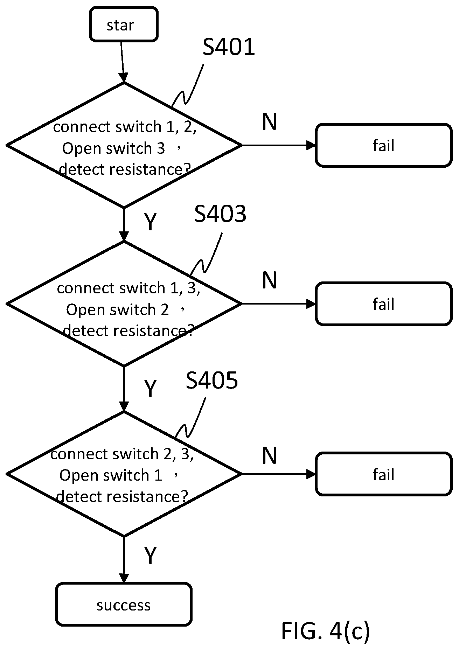

When the switch circuit is configured as shown in FIG. 4(b), the method for testing the attachment of the piezoelectric patch 20 is processed as shown in FIG. 4(c). First, in step S401, the first switch SW1 (switch 1) and the second switch SW2 (switch 2) are connected, and the third switch SW3 (switch 3) is disconnected to check whether the resistance is detected. If not, it means that the attachment is not good; if yes, step S403 is performed, the first switch SW1 (switch 1) and the third switch SW3 (switch 3) are connected, and the second switch SW2 (switch 2) is disconnected to check whether the resistance is detected or not. If negative, the attachment is not complete; if positive, step S405 is processed, the first switch SW2 (switch 2) and the third switch SW3 (switch 3) are connected, and the first switch SW1 (switch 1) is disconnected to check whether resistance is measured or not. If negative, it means that the attachment fails. If positive, the attachment is completed.

Another embodiment of the stethoscope 101 includes a microphone having a capacitive sensor. The capacitive sensor employs an ultra-thin material as a diaphragm (for example, 30 µm thick glass), the capacitive sensor is plated with a conductive material, and the diaphragm is sealed with a circuit board by means of a sealant to form a resonance chamber or cavity. The purpose is to use the sound of the heartbeat to vibrate the diaphragm, causing a change in capacitance between the diaphragm and the circuit board. The stethoscope 101 captures this change to record the heartbeat.

The capacitive sensors contact with the body, entirely. A pressure sensor is arranged on the sound isolating ring of the capacitive sensor. The pressure sensor includes piezoelectric, capacitive, resistive and other type sensors. It is placed between the sound isolating ring and the circuit board or under the circuit board. Basically, the pressure sensor generates a corresponding pressure signal while it is pressed. Therefore, the pressure sensor determines the fitness degree between the wearable capacitive sensor and the user based on the sensed pressure signal.

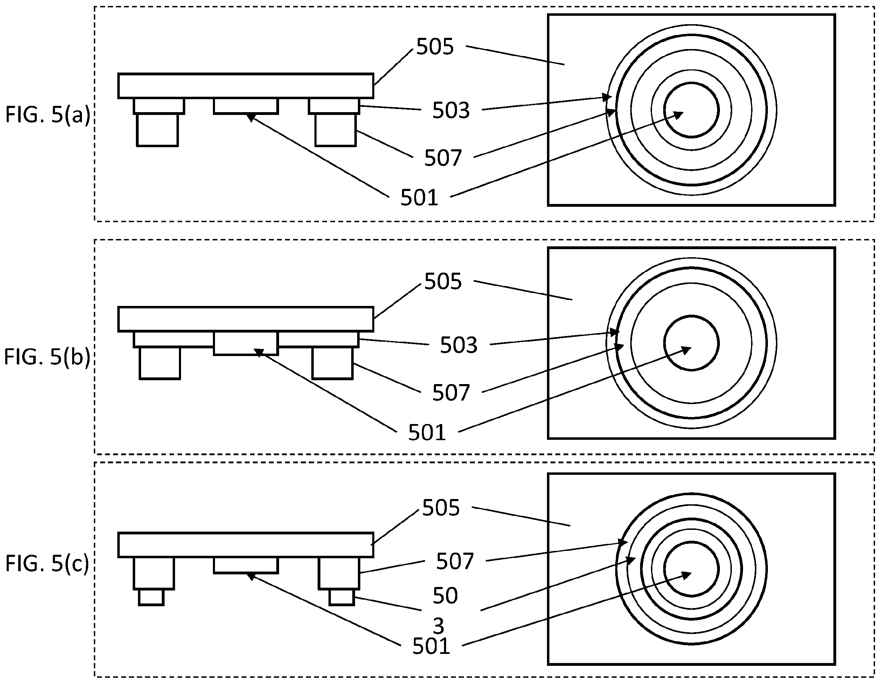

Please refer to FIG. 5(a)-(c), they respectively show several embodiments of the pressure sensor configuration, each figures shows the side view at left side, and top view at the right side. In FIG. 5(a)-(b), the pressure sensor 503 is disposed between the circuit board 505 of the capacitive sensor 501 and the sound isolating ring 507; alternatively, please refer to FIG. 5(c), the pressure sensor 503 is disposed under the sound isolating ring 507.

The capacitive sensors may face problems, such as poor response at low frequencies, large ambient noise and other technical difficulties, based on the issues, the wearable heart sound collecting device integrates the piezoelectric sensor structure as shown in Figures 3-4 and the capacitive sensor disclosed in Figure 5. The integrated wearable heart sound collecting device will be discussed in the subsequent paragraphs.

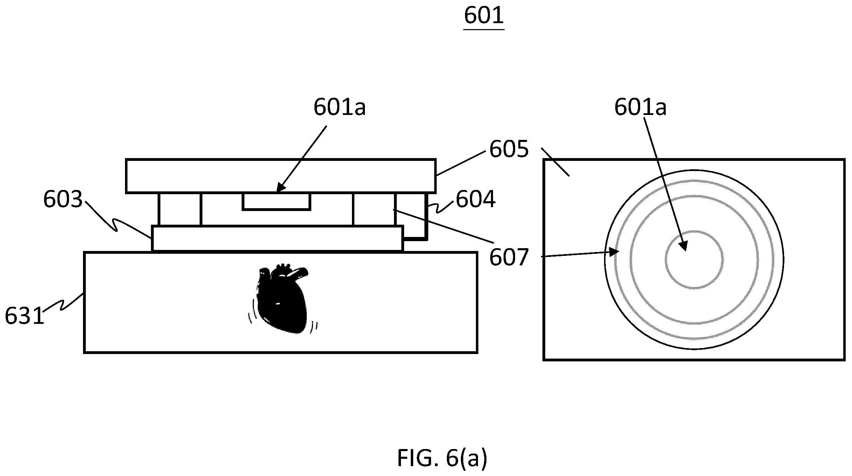

FIG. 6(a) shows the physiological sound collecting device (stethoscope) 601 according to an embodiment of the present invention, which includes a diaphragm 601a, on which a conductive material is plated, and the diaphragm 601a is packaged with a plastic frame (sound isolating ring) 607 and a circuit board 605. The sound isolating ring 607 and the circuit board 605 form the resonance cavity. The sound isolating ring 607, the circuit board 605 combines with the diaphragm 601a in the resonance cavity to form a microphone structure. A piezoelectric sensor 603 is disposed under the sound isolating ring 607 and is electrically connected to the circuit board 605 via a conductive line 604. By contacting with human skin 631, it is used to measure a voltage signal generated by vibration. The capacitive sensor is poor respondence to low-frequency signals, such as the third and fourth heart sounds frequencies are around 20 Hz. Therefore, the piezoelectric sensor 603 is utilized as another diaphragm to improve the situation.

In one embodiment, the piezoelectric sensor 603 is formed on the flexible substrate (see FIG. 2) and is in the form of patch. A plurality of electrodes is disposed at the bottom of the flexible substrate to determine whether the attachment is completed or not. In one embodiment, the stethoscope (heart sound collecting device) 601 is directly attached to the user's skin 631 above the heart by the patch to measure heart sound signals.

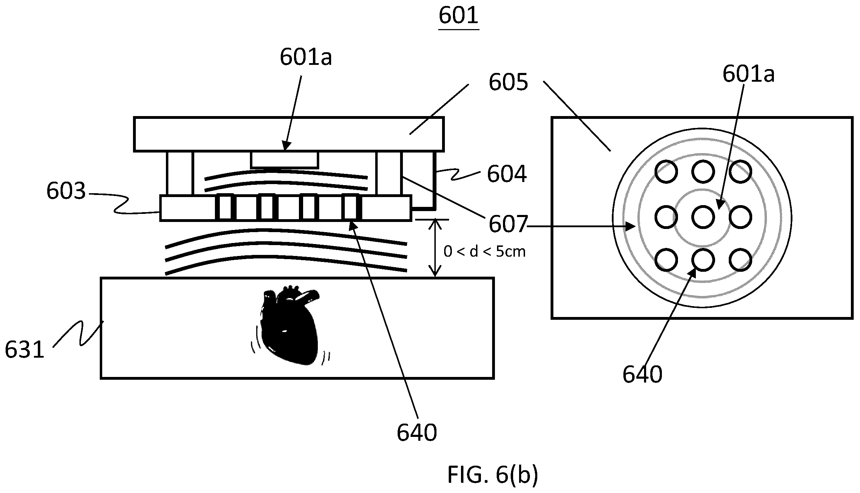

FIG. 6(b) shows the physiological sound collecting device (stethoscope) 601 according to another embodiment of the present invention, it includes a diaphragm 601a, on which a conductive material is plated, and the diaphragm 601a is packaged with a plastic frame 607 and a circuit board 605. A piezoelectric sensor 603 is disposed under the sound isolating ring 607 and is electrically connected to the circuit board 605 by the conductive line 604. In the case, holes 640 are formed in the piezoelectric sensor 603. This design allows sound to pass through the holes, so it does not need to contact with the human body under measuring. In one embodiment, the size of the hole in the piezoelectric sensor 603 is in the range of 10 µm to 1000 µm. In one embodiment, the integrated physiological sound collecting device (stethoscope) 601 is designed with holes in the piezoelectric sensor 603, wherein the distance d between the piezoelectric sensor 603 and the human skin 631 is in the range of 0<d<5 cm.

The above-mentioned sensor is employed to receive heart beating signals. The integrated wearable heart sound collecting device receives heart beat signals through the capacitive sensor and the piezoelectric sensor, respectively. In the integrated wearable heart sound collecting device, the circuit board includes amplifiers, filters, power management system, identification system, Bluetooth and processor.

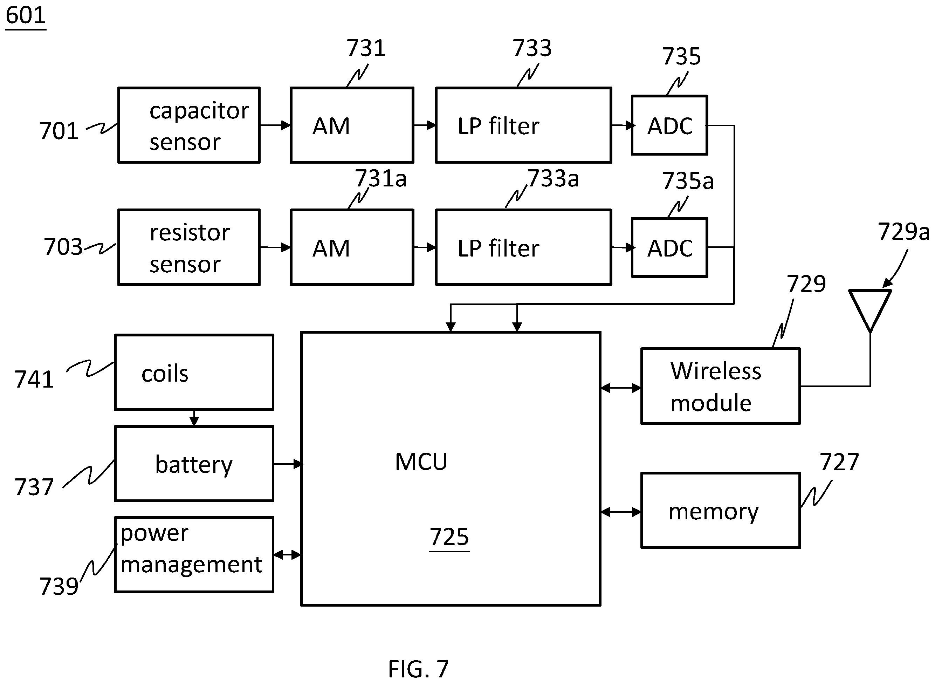

FIG. 7 shows a functional block diagram of the physiological sound collecting device 601 which obtains sound signals from the human body by the capacitive sensor 701 and the piezoelectric sensor 703, respectively. The wearable physiological sound collecting device 601 includes a microprocessor, a storage unit and a wireless transmission module to receives data, transmit data, and executes software applications.

The microprocessor 725 can be a microcontroller, a digital signal processor (DSP), an application specific integrated circuit (ASIC), a programmable logic circuit or other digital data processing device that executes instructions to perform processing operations. The microprocessor 725 can execute various application programs stored in the storage unit, including executing firmware algorithms.

The storage unit 727 may include a read-only memory (ROM), a random-access memory (RAM), an electrically erasable programmable ROM (EEPROM), a flash memory or any memory used in a computer.

The wireless transmission module 729 is connected to the antenna 729a to send output data and receive input data via wireless communication channels. The wireless communication protocol includes WiFi, Bluetooth, RFID, NFC, 3G/4G/5G/6G or any other future wireless communication protocol.

The signals fetched by the capacitive sensor 701 and the piezoelectric sensor 703 are amplified by the first and second amplifiers 731 and 731a respectively, followed by filtering by the first and second low-pass filters 733 and 733a. The filtered heart sound signal is converted into a digital signal through the first and second analog-to-digital converters (ADC) 735 and 735a, and then processed by the microprocessor 725 to obtain a de-noising and stable heart sound signal. The microprocessor 725 stores the de-noising and stable ECG signals, body sound signals in a storage unit by instructions or programs, or send the signals to an external device, such as a smart phone, tablet, for further analysis via a wireless transmission module 729.

The battery pack 737 provides power for the wearable physiological sound collecting device 601 and cooperates with the power management unit 739 to optimize power utilization. In addition, the battery pack 737 can also be wirelessly charged via a charging coil 741.

In one embodiment, the microprocessor 725, the storage unit 727, the wireless transmission module 729, the amplifiers 731, 731a, the low-pass filters 733, 733a, the analog-to-digital converters (ADC) 735, 735a, and the power management module 739 can be integrated into a single circuit module.

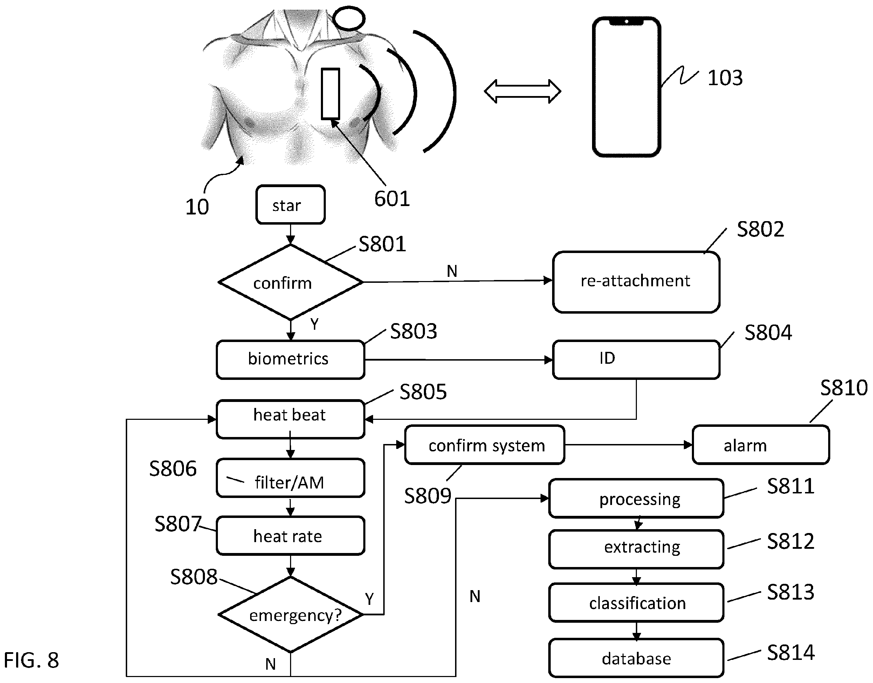

According to Figures 2-7 and related embodiments, the present invention provides the wearable sound detection system as shown in Figure 1, which includes the wearable physiological sound collecting device 601 and a mobile device (smartphone or tablet computer, etc.) 103 to provide real-time health care monitoring. In the event of an emergency, an alarm is issued through the mobile device 103 to seek help in time. The execution process of establishing the system is shown in Figure 8.

First, step S801 is to confirm whether the attachment of the physiological sound collecting device 601 to the user's body 10 is completed or not. If the attachment fails, the mobile device 103 will notify (step S802) the user. In step S803, the biometrics of the user are collected; subsequently, in step S804, the collected biometrics are used to identity the user; after the identity is confirmed, the next step S805 is performed, in S805, the physiological sound collecting device 601 is used to collect the (heartbeat) signal of the user; then, in step S806, the sound signal is filtered and amplified; in step S807, a preliminary heart rate analysis is performed; after the heart rate analysis, step S808 is performed to determine whether there is an emergency situation. If an issued is detected (such as no heartbeat is measured...), the device will immediately connect to the mobile device 103 and to compare with other sensors (step S809). If a critical situation is confirmed, the mobile device 103 will immediately issue an alarm (step S810); if it is normal, the heart sound signal will be sent to the mobile device 103 for further signal processing and the device keeps to collect signals, step S811. After receiving the normal heart sound signal, the mobile device 103 performs signal processing, such as filtering, wavelet analysis, Fourier transform, etc., and thereafter to extract the characteristics of the heart sound signal (Step S812). Then, in Step S813, the characteristics are used to compare with the database and the previous data by artificial intelligence (AI), and the conditions are checked to confirm whether there are abnormal signs; in Step S813, the results of the above comparison and condition classification are sent to the database for subsequent comparison reference, they are also submitted to the medical care unit for medical suggestions after reviewing.

In one embodiment, the database can be a cloud database in a cloud server. The normal heart sound signals and abnormal heart sound signals are classified by AI comparison and AI classification algorithm installed in the mobile device 103. The AI algorithm may include a series of steps: pre-filtering and normalizing the input heart sound signal, extracting time domain and frequency domain features, and outputting classification results using a convolutional neural network (CNN) model. In one embodiment, the pre-filtering and normalization processing of the heart sound signal is performed by software application.

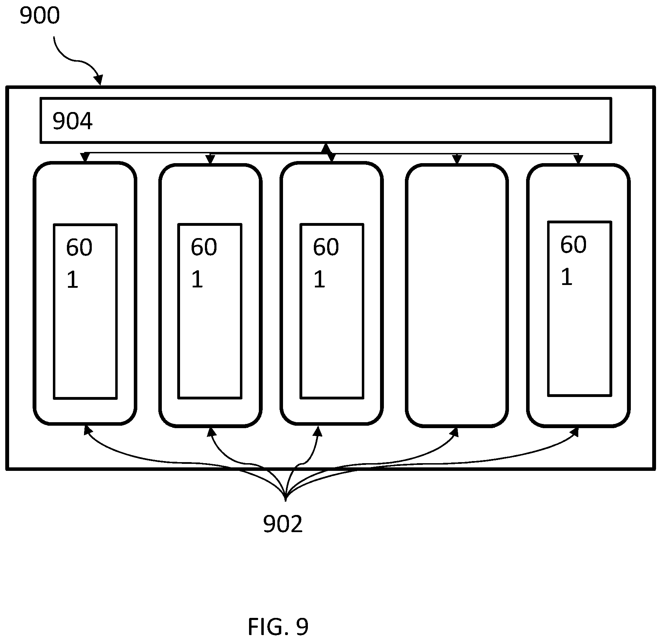

FIG. 9 is a schematic diagram of wireless charging of the present invention. A stethoscope charging device 900 has a battery 904 and a plurality of wireless transmitting coils 902, for example, a plurality of stethoscopes 601 is placed on the stethoscope charging device 900. Wireless charging (also known as inductive charging) utilizes inductive coupling to transmit energy from the stethoscope charging device 900 to the physiological sound collecting device 601, and thereby charging the battery 737 of the physiological sound collecting device 601 through the charging coil 741. The stethoscope charging device 900 transmits energy by inductive coupling, no wire connection is required between the two devices. The following wireless specification could be used, for example, Qi standard of the Wireless Power Consortium (WPC), AirFuel Resonant (A4WP standard) and AirFuel Inductive (PMA standard) from the AirFuel Alliance (AFA). No power contact design avoids the risk of electric shock. When implanting in medical devices, wires are not necessary. The stethoscope charging device 900 of the present invention may charge multiple physiological sound collecting device 601 simultaneously, no multiple chargers are required, and no more hassles of multiple wires tangled together.

The stethoscope charging device 900 has built-in control circuits required for power transmission and reception, and does not require an external microcontroller. It is suitable for long time wearing and it has larger battery capacity. In one embodiment, a high frequency band of 13.56 MHz is introduced to support contactless communication, for example, Near Field Communication (NFC) standard.

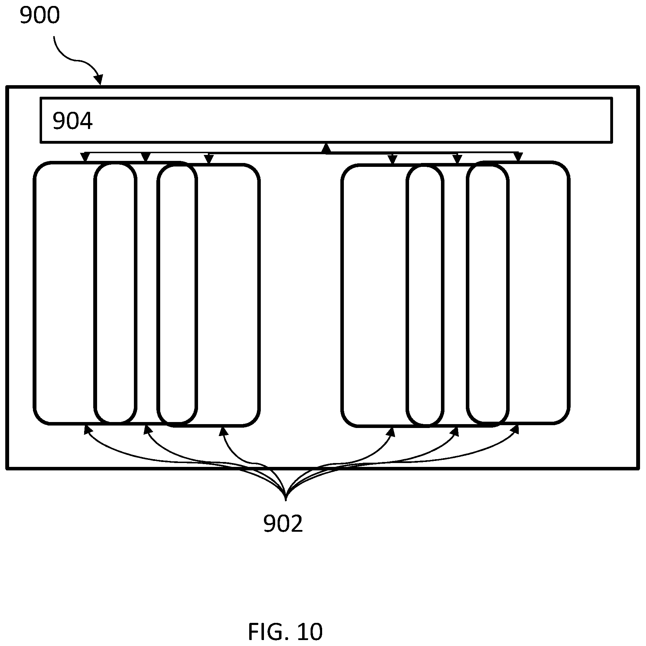

In another embodiment, the multiple wireless transmitting coils 902 are partially overlap, which is beneficial to reduce the misalignment of the physiological sound collecting device (stethoscope) 601 on the charging board. When the stethoscope 601 is placed on the charging board, even if there is a position misalignment, it can still be effectively charged, please refer to Figure 10.

Preferably, the heart rate, electrocardiogram and blood oxygen saturation level can be directly measured by sensors, while blood pressure is measured through electrocardiogram (ECG) and pulse wave (PPG). The pulse transit time (PTT) signal could be obtained through the blood oxygen saturation level.

Heart condition reveals lots of valuable information about the human body. A general medical equipment monitors the heart rate and activity by measuring electrophysiological signals and electrocardiogram (ECG). Electrodes are connected to the body to measure the heart signal induced by electrical activity in heart tissue. In addition, as the heart beats, a pressure wave passes through the blood vessels. This pulse wave slightly changes the diameter of the blood vessels. Therefore, in addition to ECG, the pressure wave can be used to measure the photoplethysmography (PPG) of the blood by a light source and a photoelectric sensor. Therefore, it is also called the PPG signal. It is an optical technology to fetch the heart condition information without measuring bioelectric signals. PPG technology is mainly used to measure blood oxygen saturation (SpO2) without employing bioelectrical signals. With the PPG, the heart rate monitor is integrated into a wearable device to achieve real-time detection applications.

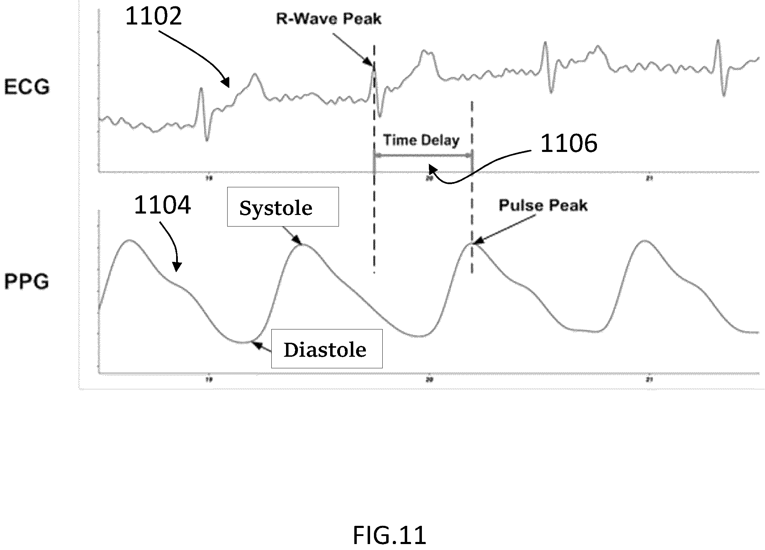

Referring to FIG. 11, the pulse wave transit time (PTT) 1106 is obtained by comparing the pulse wave signal (PPG) 1102 with the electrocardiogram signal (ECG) 1104. According to the individual physiological characteristic points of the pulse wave signal (PPG) 1102 and the electrocardiogram (ECG) 1104, the peak value of the electrocardiogram (ECG) 1102 comes from the contraction of the ventricle, while the peak value of the pulse wave signal (PPG) 1104 comes from the contraction of the blood vessel. Thus, the transmission time from the heart to the measuring site is obtained, it refers to the pulse wave transit time (PTT) 1106. Specifically, a widely used method for obtaining the pulse wave transit time (PTT) includes steps to measure the time delay between the R peak value (the position indicated by the dotted line) of the electrocardiogram signal (ECG) 1102 and the characteristic points of the pulse wave signal (PPG) 104, such as the pulse wave signal (PPG) peaks (dashed lines indicate positions). The time delay refers to the required time for the pulse wave travels from the proximal end to the distal end. Similarly, the pulse transit time (PTT) can also be obtained by comparing the pulse wave signal (PPG) 1102 with the heart sound signal.

Since the speed of pulse wave transmission is directly related to the blood pressure, when the blood pressure is high, the transmission of the pulse wave is fast, and vice versa. Therefore, the pulse is obtained by the electrocardiogram signal (ECG) 1102 and the pulse wave signal (PPG) 1104. The pulse wave transmitting rate is obtained by considering some body parameters (such as height and weight). The systolic and diastolic pressures of the human pulse can be estimated by the established characteristic equation to achieve the purpose of non-invasive and continuous real-time blood pressure measurement.

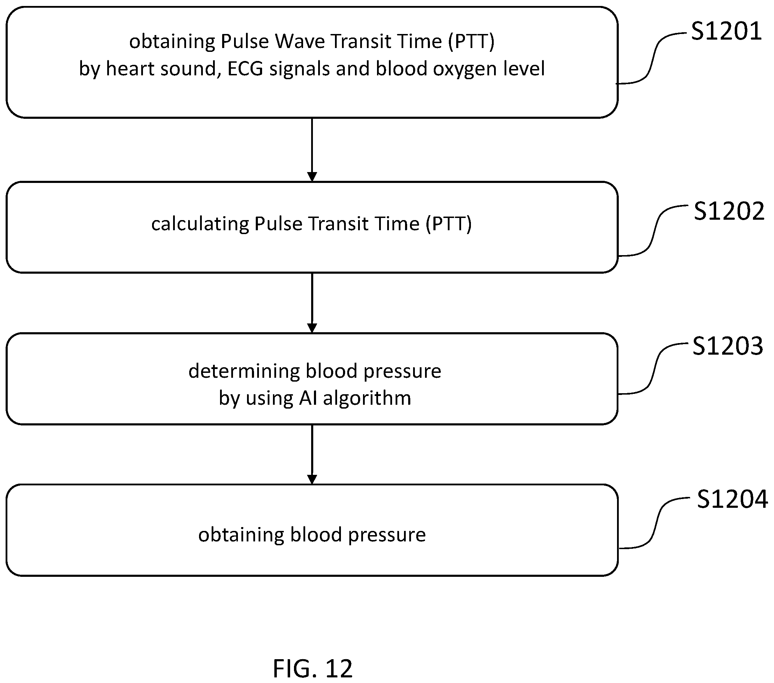

FIG. 12 shows a method 1200 for measuring blood pressure disclosed in the present invention, which includes performing the following steps: first, in step S1201, a physiological sound sensing device 1302 of the wearable physiological signal sensing system 1300 (see FIG. 3A) is provided. The ECG sensing device 1304 (e.g., ECG patch (ECG+, ECG-)) and the blood oximeter 1306 (i.e., pulse wave signal sensing device) respectively sense a user's heart sound signal, ECG signal, and blood oxygen level. The blood oxygen level includes oxygen saturation and pulse wave signal (e.g., fingertip pulse wave signal); subsequently, in step S1202, the blood oxygen signal (pulse wave signal) is compared with the electrocardiogram signal, alternatively, the heart sound signal is compared with the blood oxygen signal (pulse wave signal). The blood oxygen signal (pulse wave signal) is calculated to obtain the pulse wave transit time (PTT). Then, in step S1203, the user's blood pressure is determined by an artificial intelligence (AI) algorithm. In step S1204, the blood pressure value is fetched based on the result of the algorithm.

According to an embodiment of the present invention, the above-mentioned artificial intelligence (AI) algorithm for predicting the blood pressure is based on the pulse wave transit time and pulse wave transit velocity. This method employs a multivariate linear model established by the time domain characteristics of the accelerated pulse wave, the pulse wave transit time (PTT) and the user's height parameters.

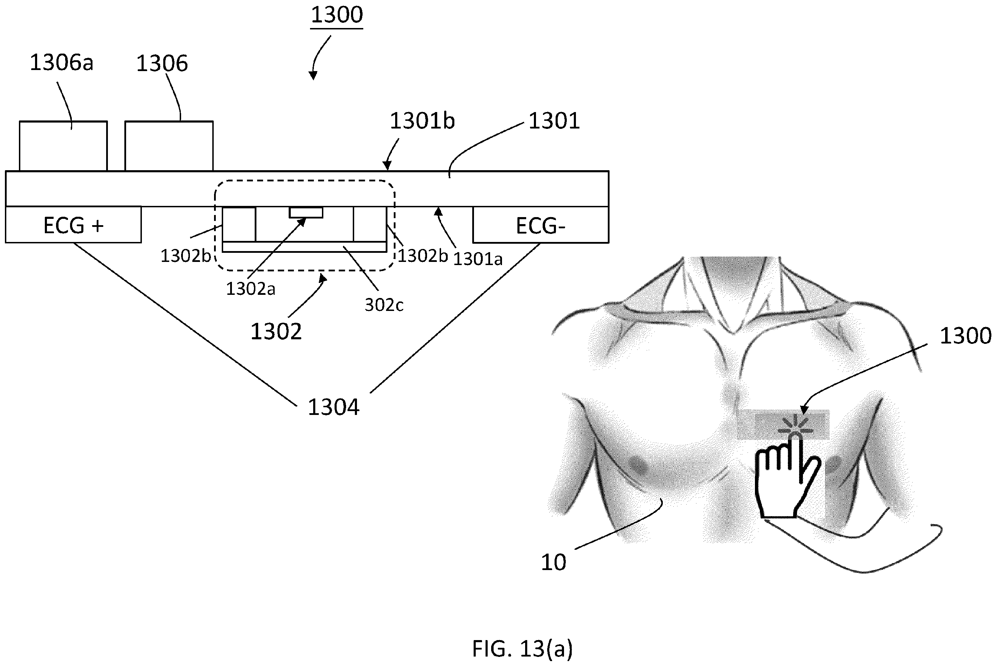

FIG. 13(a) is a cross-sectional view of the wearable physiological signal sensing system 1300 according to an embodiment of the present invention, which includes a circuit board 1301 having a first surface (lower surface) 1301a, on which a stethoscope 1302 and a plurality of ECG patches 1304 are provided. A blood oximeter 1306 is provided on the second surface (the upper surface) 1301b of the circuit board 1301. For the convenience of wearing, the wearable physiological sensing system 1300 is attached to the chest of the user 10 in the form of patch. When the wearable physiological sensing system 1300 is used, a plurality of ECG patches 1304 (eg, a pair of ECG+ and ECG- electrodes) are attached to the chest skin of the user 10.

**In** one embodiment, the stethoscope 1302 includes a diaphragm 1302a, on which a conductive material is plated. The diaphragm 1302a is packaged with a plastic frame (sound isolating ring) 1302b and the circuit board 1301. Wherein the sound isolating ring 1302b and the circuit board 1301 form a resonance cavity. The microphone structure is constructed by combining the resonance cavity with the diaphragm 1302a, a piezoelectric sensor 1302c is disposed under the sound isolating ring 1302b and is electrically connected to the circuit board via conductive lines (not shown), through contact with the user's skin, it is used to measure the voltage signal generated by vibration. Namely, the piezoelectric sensor 1302c can be used as another diaphragm to assist the capacitive sensor in poor responding to low-frequency signals such as the third and the fourth heart sounds (around 20 Hz).

The components of the microphone structure include capacitive sensors, which use an ultra-thin material as a diaphragm 1302a (for example 30 µm thick glass), the diaphragm 1302a is coated with a conductive material. The diaphragm 1302a is packed with the circuit board 1301 by the frame glue 1302b, thereby forming a resonance cavity. The purpose of the structure is to vibrate the diaphragm 1302a using the sound of the heartbeat, thereby causing a change in capacitance between the diaphragm 1302a and the circuit board 1301. The stethoscope 1302 (heart sound capturing device) captures this change to record the heart sound signal.

The piezoelectric sensor 1302c is mainly composed of a piezoelectric material layer (for example, polyvinylidene fluoride (PVDF) polymer piezoelectric film, lead zirconate titanate (PZT) and other materials), and its upper and lower surfaces are plated with conductive metal (e.g., aluminum (Al), copper (Cu), etc.). Leads are out from each of the upper and lower metal layers and the leads connected to the circuit board. They are used to measure the voltage signal generated by vibration. In one embodiment, the thickness of the piezoelectric sensor is less than 50 µm.

In one embodiment, the stethoscope 1302 is used to measure the sound signal of the user 10. In one embodiment, the plurality of ECG patches 1304 include at least two electrodes, namely ECG+ and ECG-, for measuring the user's ECG signals. In one embodiment, the oximeter 1306 is used to measure the PPG signal and blood oxygen saturation (SpO2) of the user 10. According to the embodiment of the present invention, the oximeter 1306 includes an infrared light source (infrared light LED), a red-light source (red light LED) and a photoreceptor for sensing the fingertip PPG signal of the user 10, namely, the fingertip pulse wave.

As shown in FIG. 13(a), the stethoscope 1302 and the plurality of ECG patches 1304 in the wearable physiological signal sensing system 1300 are attached to the chest of the user 10, wherein the stethoscope 1302 and the plurality of ECG patches 1304 are used to capture the sound signals and electrocardiogram signals respectively. The operation method of the wearable physiological signal sensing system 1300 includes: (1) attaching the wearable physiological sensing system 1300 to the chest of the user 10 to obtain the heart sound and electrocardiogram information of the user 10. The user 10 continuously monitors the PCG signals and ECG signals. (2) The user 10 presses the oximeter with his finger to obtain the pulse wave signal (PPG) and blood oxygen level (non-continuous monitoring, where PPG signals and blood oxygen saturation (SpO2) are monitored only when information is required). (3) obtaining the pulse wave transit time (PTT) from the heart to the finger of the user 10, and calculating blood pressure by an artificial intelligence (AI) algorithm, followed by fetching the blood pressure of the user 10 (non-continuous monitoring).

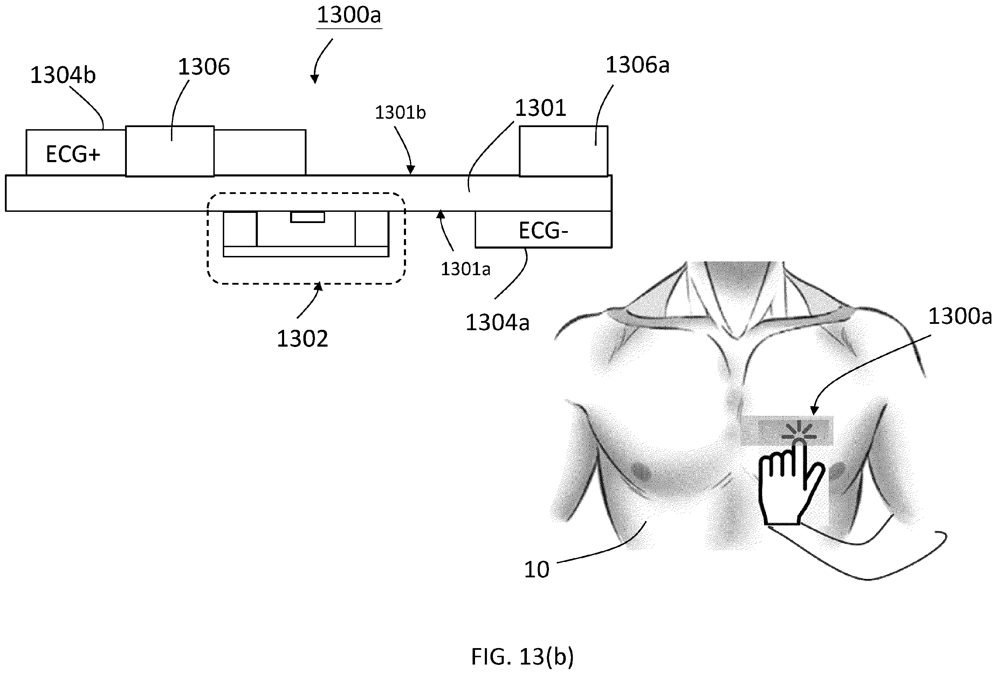

FIG. 13(b) is a cross-sectional view of the wearable physiological signal sensing system 1300a according to another example of the present invention, which includes a circuit board 1301 having a first surface (lower surface) 301a. A stethoscope 1302 and a first ECG patch 1304a are provided on the first surface (lower surface) 1301a. A blood oximeter 1306 and a second ECG patch 1304b (both are integrated) are formed on the second surface (opposite side of the first surface, i.e., the upper surface) 1301b of the circuit board 1301. In one embodiment, the structure of the stethoscope 1302 is the same as that of the stethoscope described in FIG. 13(a), and will not be described in detail herein. In one embodiment, the stethoscope 1302 is used to measure the heart sound signal of the user 10. In one embodiment, the first ECG patch 1304a and the second ECG patch 1304b are one of the positive and negative electrodes (i.e., ECG+ and ECG-), and both are respectively disposed on the opposite side of the circuit board 301 to measure the ECG signals of the user 10. In one embodiment, the oximeter 1306 is used to measure the PPG signal and blood oxygen saturation (SpO2) of the user 10.

As shown in FIG. 13(b), the stethoscope and the first ECG patch 1304a in the wearable physiological sensing system 1300a are attached to the chest of the user 10, and the second ECG patch 1304b and the oximeter are integrated and disposed on the opposite side of the stethoscope 1302. The stethoscope 1302 is used to capture heart sound signals. The first and second ECG patches (1304a, 1304b) are utilized to capture ECG signals, and the oximeter 1306 is used to capture PPG signals and blood oxygen saturation (SpO2). The operation method of the wearable physiological sensing system 1300a includes: (1) attaching the wearable physiological sensing system 1100 to the chest of the user to obtain the heart sound information of the user 10; (2) the user 10 presses the oximeter and one of the ECG electrodes (i.e., one of the first ECG patch or the second ECG patch) with a finger to obtain blood oxygen level, fingertip pulse wave (PPG) signal and ECG signal (non-continuous monitoring, where ECG signal, PPG signal and blood oxygen saturation (SpO2) are monitored only when information is needed); (3) PTT from the heart to the finger of the user 10 is obtained, and the blood pressure of the user 10 is determined by the artificial intelligence (AI) algorithm (non-continuous monitoring).

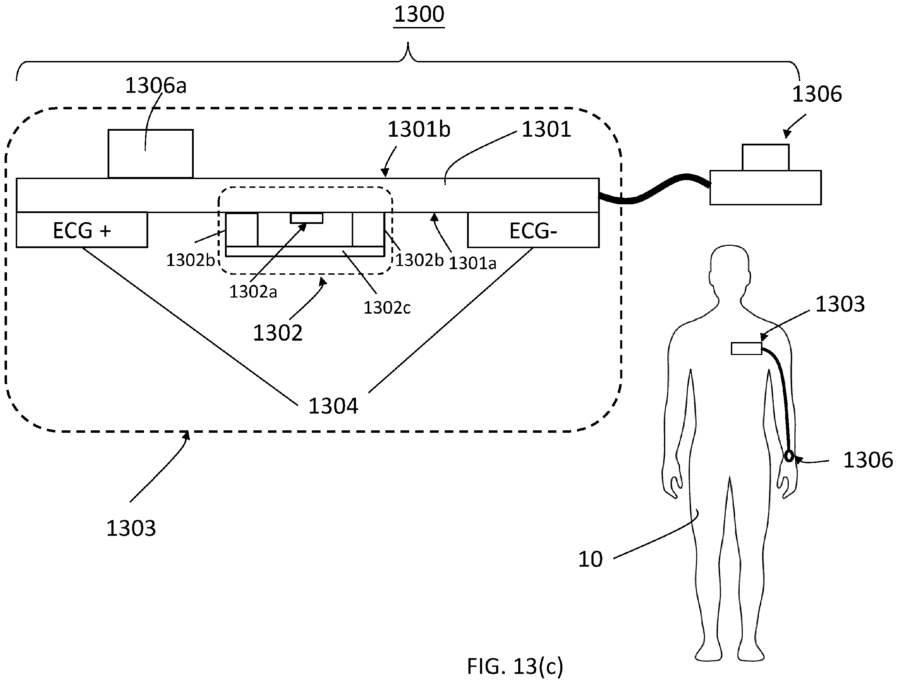

FIG. 13(c) is a cross-sectional view of the wearable physiological signal sensing system 1300 according to another embodiment of the present invention, wherein the oximeter 1306 and the electrocardiogram stethoscope device 1303 are separated and coupled by a coaxial cable or metal line for electrical connection. The electrocardiogram stethoscope device 1303 includes a plurality of ECG patches 1304, a stethoscope 1302 and a circuit board 1301. In this embodiment, the plurality of ECG patches 1304 and the stethoscope 1302 of the ECG stethoscope device 1303 are disposed on the same side of the circuit board 1301. The structure of the stethoscope 1302 is the same as that of the stethoscope described in FIG. 13(a) and the detail description is omitted here.

In one embodiment, the stethoscope 1302 is used to measure the heart sound signal of the user 10; the plurality of ECG patches 1304 having positive and negative electrodes (i.e., ECG+ and ECG-) are used for measuring ECG. The oximeter 1306 is employed to measure the PPG signal and the blood oxygen saturation (SpO2) of the user 10.

Referring to FIG.1 3C, the ECG stethoscope device 1303 is attached to the chest of the user 10 when in use, and the oximeter 1306 can be placed on body parts such as, but not limited to, the user's finger, palm, wrist, arm, thigh, calves, ankles, toes, ears, forehead or cheeks etc. according to actual needs.

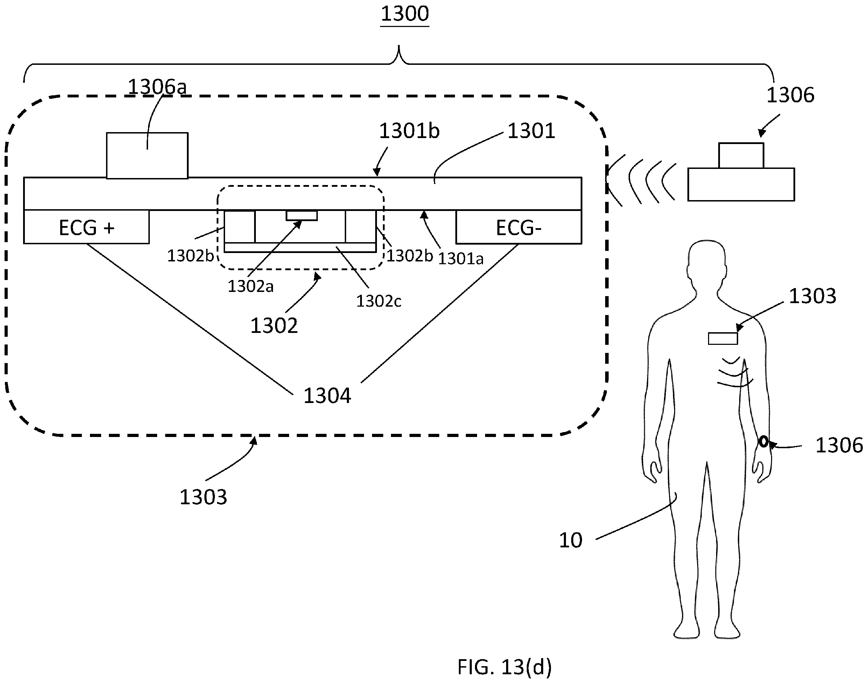

FIG. 13(d) shows a cross-sectional view of the wearable physiological signal sensing system 1300 according to another example of the present invention, wherein the oximeter 1306 and the electrocardiogram stethoscope device 1303 are separated and coupled by a wireless connection including Bluetooth, Wifi, and so on. The structure and usage of the blood oximeter 306 and the stethoscope device 1303 are the same as the embodiment of FIG. 13(c), and the detail description is omitted.

According to an embodiment of the present invention, the blood oximeter 1306 shown in FIGS. 13(a)-13(d) includes an infrared light source (infrared light LED), a red-light source (red light LED) and a photoreceptor for sensing the user's 10 fingertip PPG signal, that is, fingertip pulse wave.

According to an embodiment of the present invention, the circuit board 1301 can be disposed on a flexible substrate. The above-mentioned flexible substrate includes a fabric, polyimide (PI) or polyethylene terephthalate (PET).

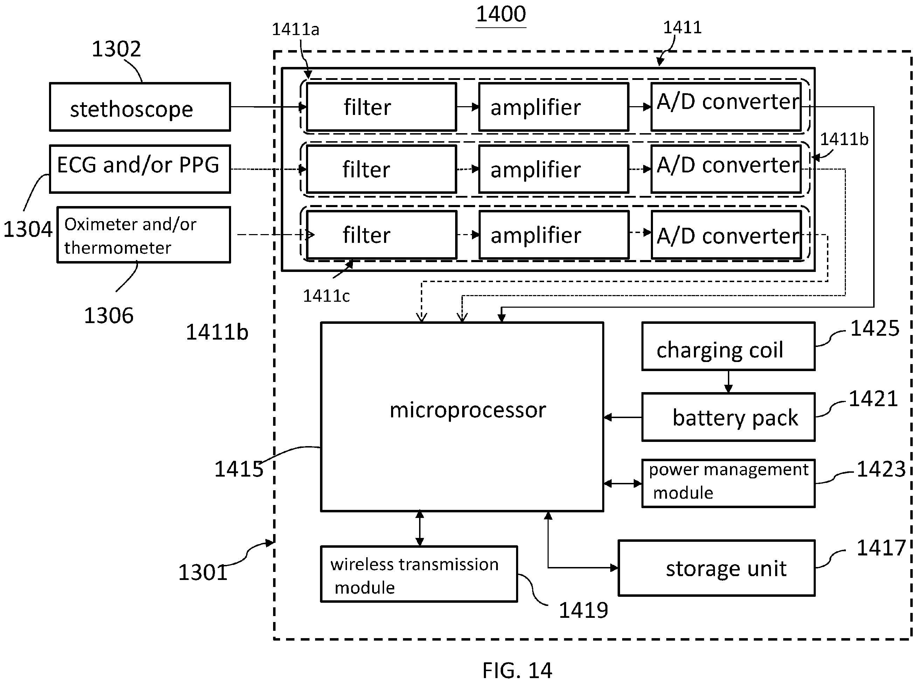

FIG. 14 is a functional block diagram of the wearable physiological signal sensing system 1400, wherein a stethoscope 1302 can detect heart sound signals from the user's body; a pair of electrocardiogram electrodes (ECG electrodes), i.e., an electrocardiogram patch 1304, can detect ECG signals from the user's body; the oximeter 1306 is used to detect PPG signals and blood oxygen saturation (SpO2). The heart sound signal, electrocardiogram signal and PPG signal are filtered, amplified and converted between analog/digital signals by corresponding signal preprocessing channels 1411a, 1411b and 1411c in the signal preprocessing circuit 1411 to obtain preprocessed digital signals, heart sound signals, ECG signals and PPG signals. In one embodiment, each pre-processing channel includes a filter, a signal amplifier, and an analog/digital (A/D) converter.

The microprocessor 1415 can store the above-mentioned stable and background noise-free cardiac sound signal, ECG signal and PPG signal in the storage unit 1417 through instructions or programs, or send said signals to an external mobile device by a wireless transmission module 1419 for further analysis. The mobile device may be a smart phone. The microprocessor 1415 compares the ECG signal with the PPG signal or the heart sound signal with the PPG signal for computing the pulse wave transit time (PTT) by the two signals, and thereby achieving the continuous blood pressure from the PTT. According to an embodiment of the present invention, the microprocessor 1415 performs blood pressure prediction by the artificial intelligence (AI) algorithm mentioned above.

The battery pack 1421 provides power for the integrated cardiac sound and electrocardiographic signal sensing device 400, and cooperates with the power management module 1423 to optimize power usage. The battery pack 1421 could be wirelessly charged by a charging coil 1425.

In one embodiment, the microprocessor 1415, storage unit 1417, wireless transmission module, signal preprocessing circuit 1411, and power management module 1421 can be integrated into a single system circuit board, such as the circuit board 1301 shown in FIG. 13(a) and 3B.

In one embodiment, the user may view the collected electrocardiogram (ECG), phonocardiogram (PCG) and PPG signals through an external computing device, and these data are further analyzed and compared by the external computing device which is selected from a smart phone, a tablet computer or a cloud server.

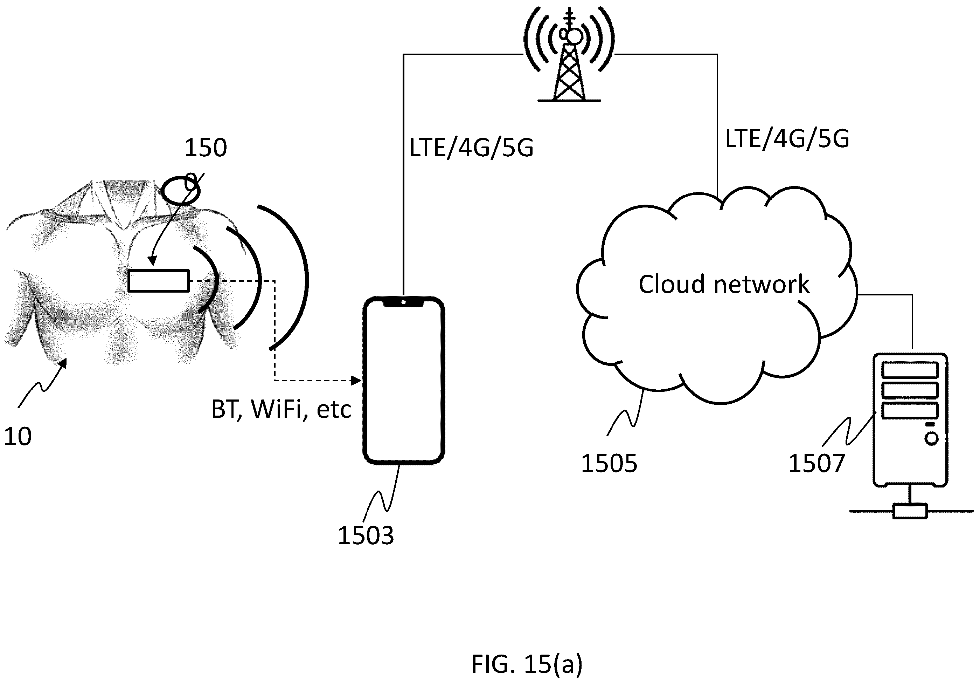

As shown in FIG. 15(a), according to one embodiment of the present invention, the wearable physiological signal sensing system 1500 includes a circuit board 1301 (refer to FIG. 13(a)) having a first surface (lower surface) 1301a of the circuit board 1301. A stethoscope 1302 and a plurality of ECG patches 1304 are provided. A blood oximeter 1306 is provided on the second surface 1301b of the circuit board 1301, the second surface 1301b refers to the opposite side of the first surface, i.e., the upper surface. When it is worn on the user 10, it may communicate with the mobile device 1503, such as a smart phone or a tablet computer. The physiological data (including heart sound signals, electrocardiogram signals, blood oxygen signals and blood pressure signals) are collected by the wearable physiological signal sensing system 1500. These data may be transmitted by wireless, for example, Bluetooth and WiFi. The mobile device 1503 uploads the data to the cloud server 1507 via the cloud network 1505. In the cloud server 1507, the data will be stored in the cloud database. The system further includes an application installed in the mobile device 1503, wherein the application includes instructions for receiving and sending data between the wearable physiological signal sensing system 1500, the mobile device 1503 and the cloud server 1507. The above application can be operated on an Android, Windows 10 or iOS operating system platform, and can upload the collected relevant data/signals, such as electrocardiogram signals, heart sound signals, blood oxygen signals and their waveforms, to the cloud server 1507 for processing. The data is stored, analyzed and processed by data analysis and feature extraction algorithms to generate an evaluation report for medical advice accordingly.

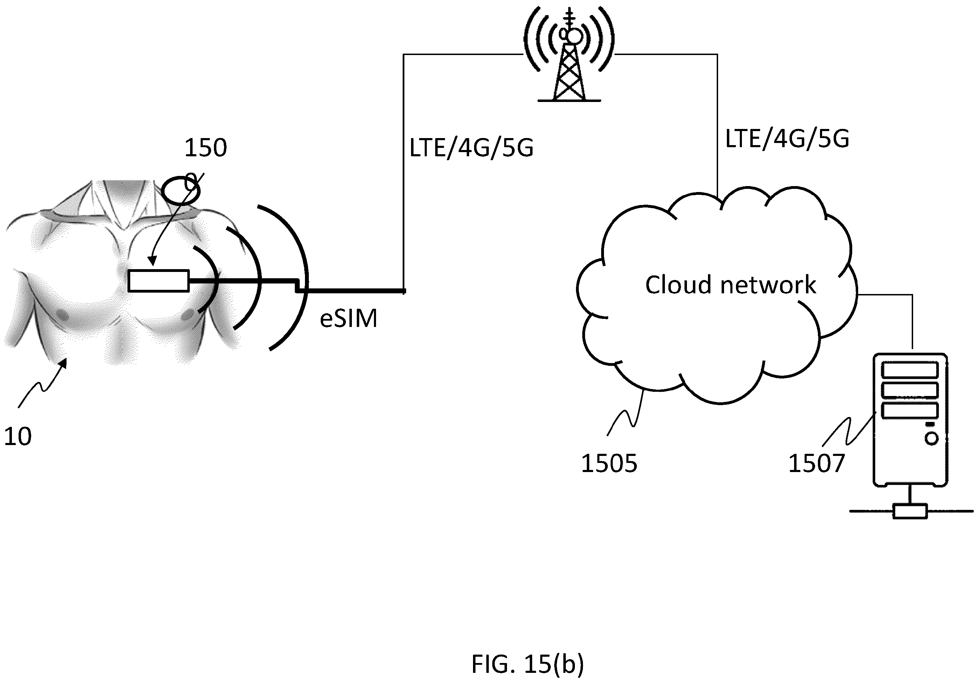

Considering that the elderly are not familiar with using mobile devices, such as smartphones, tablets, referring to FIG. 15(b), according to another embodiment of the present invention, an eSIM is embedded on the circuit board 1301 of the wearable physiological signal sensing system 1500 (also refer to FIG. 13(a)). That is, the eSIM is provided inside the wearable physiological signal sensing system 1500. The above physiological data collected by the wearable physiological signal sensing system 1500 is directly uploaded to the cloud server 1507 from the cloud network 1505 through the wireless transmission of the eSIM card. In the cloud server 1507, the data will be stored in a cloud database. The eSIM card may be configured in hardware, firmware or software form.

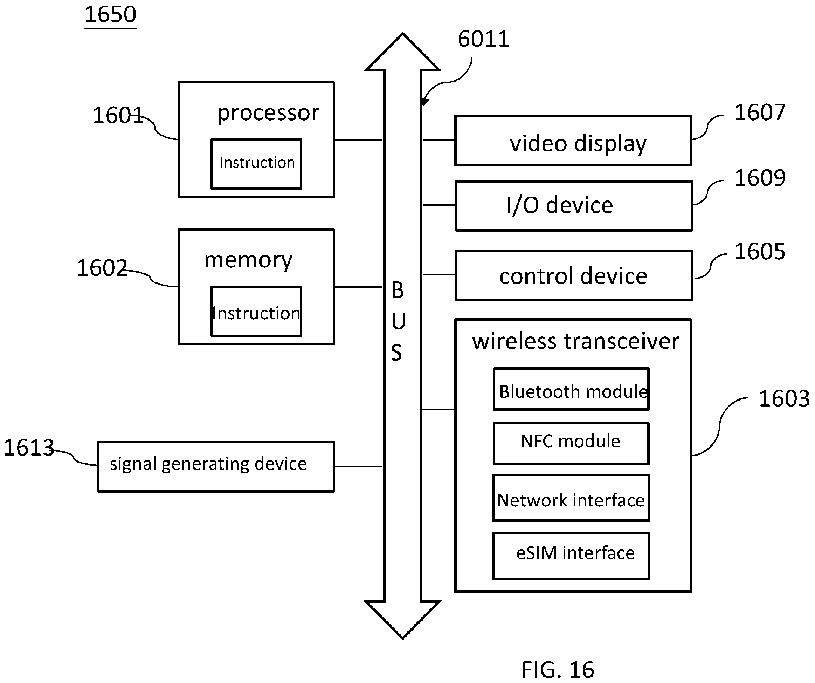

FIG. 16 shows a functional block diagram of a processing system 1650 of the mobile device 1503 or the cloud server 1507. Specifically, the processing system 1650 refers to the processing system of the mobile device 1503 or the computing system in the cloud server 1507 (FIG. 15(a)), the cloud server 1507 (or the mobile device 1503) executes instructions to perform the processing, for example, it executes the previous said algorithm for detecting cardiac abnormalities and extracts ECG signal features. Those skilled in the art should understand that the above instructions may be stored and/or executed as hardware, software or firmware without departing from the spirit of the present invention. Furthermore, those skilled in the art should understand that the exact configuration of each processing system may be different, and the processing system 1650 shown in FIG. 16 is an example only.

In one embodiment, the mobile device 1503 is coupled to an edge computing device used to replace the cloud server 1507. The goal is to reduce the amount of computation performed remotely, thereby minimizing the amount of long-distance communication between the client and server. Edge computing brings enterprise applications closer to data sources such as IoT devices, the architecture offers benefits including faster response times and better bandwidth availability. The 5G networks and mobile edge computing enable faster and more comprehensive data analysis. Therefore, in a preferred embodiment, data such as heart sounds, electrocardiograms, and pulse waves are processed by the edge computing devices using 5G or 6G networks, if the tasks are unlikely to be processed by the edge computing devices, they will be transmitted to the external cloud computing devices. The edge computing architecture can be divided into: the "device layer" for data acquisition, the "edge layer" for real-time data processing, and the "cloud layer" responsible for secure storage and in-depth analysis. In this embodiment, each sensing device captures physiological data via a built-in sensor. In one embodiment, the collected relevant data/signals, such as electrocardiogram signals, heart sound signals, blood oxygen signals and their waveforms, are uploaded to the edge computing device, and the data is analyzed through data analysis and feature extraction algorithms. The edge layer is closest to where the data is generated and the deploying range is wider than traditional cloud servers. The edge computing has better ability to process and analyze data instantly, it significantly reduces latency.

If the data requires more in-depth analysis, the information will be uploaded to the cloud server 1507 for further analysis. Although edge computing solves the bottleneck and latency problems of cloud computing, when the edge layer determines that certain data requires more detailed analysis, it will transmit the data to the cloud layer for deeper computing and storage.

The processing system 1650 includes a processor 1601, a main memory 1602, a wireless transceiver 1603 (including a Bluetooth module, a near field communication (NFC) module, etc., and a wireless network interface), a control device 1605 (such as a keyboard and an indicator device), a video display 1607, an input/output (I/O) device 1609, and a signal generating device 1613 connected to a communication bus 6011.

The main memory 1602, the wireless transceiver 1603, the video display 1607, the input/output (I/O) device 1609, and any number of other peripheral devices are connected to the microprocessor 1601 through the bus (BUS) 6011 to exchange data with the processor 601 for application programs executed by the processor.

The wireless transceiver 1603 is connected to the antenna and is employed to send and receive signals through a wireless telecommunication channel. In one embodiment, the wireless telecommunication protocol includes WiFi, Bluetooth, RFID, NFC, 3G/4G/5G/6G, or any other future wireless communication interface. The eSIM is employed in one embodiment as well. The video display 1607 receives display data from the processor 1601 and displays images on the screen. The video display 1607 may be a liquid crystal display (LCD) or an organic light emitting diode (OLED) display.

Main memory 1602 may include non-volatile memory, such as read-only memory (ROM), which stores instructions and data required to operate the individual subsystems of the processing system and to boot the system. Those skilled in the art will appreciate that any number of memories may be used to perform this function. The main memory 1602 may also include volatile memory, such as random-access memory (RAM), which stores the software instructions executed by the processor 1601 for computing processing (such as the computing processing required by the system according to the present invention). Those skilled in the art will appreciate that any type of memory may be used as the volatile memory, and the type used in the system is a design choice to those skilled in the art.

The Bluetooth module allows the processing system 1650 to establish communication with devices such as the wearable stethoscope device 1101 based on the Bluetooth technology standard. The near field communication (NFC) module allows the integrated cardiac sound and electrocardiographic signal sensing device 1500 to establish wireless communication with another device by bringing the two devices close together or in proximity. Other peripheral devices connected to the processor 1601 include Global Positioning System (GPS) and other positioning transceivers.

Processor 1601 that executes operation instructions includes a processing unit, a microprocessor or combination thereof. The processor 1601 can execute various application programs stored in the storage unit. These applications receive user input via a touch screen or directly from a keyboard. Some applications stored in the main memory 1602 and executable by the processor 1601 may be developed by UNIX, Android, iOS, Windows, Blackberry or other platforms.

The present invention employs patches to attach the wearable physiological signal sensing system to the human skin, the required physiological signals, such as heart sounds, electrocardiogram, blood oxygen saturation level could be measured, directly. Such methods do not require the sensing system to attach to the clothing, thus, the present invention does not need to measure signals through clothing. The present invention will not be interfered by friction noise from clothing, the noise affects the accuracy of the signal. To filter out the noise, noise reduction device will be employed, which increases extra cost.

Preferably, the stethoscope may be used to receive the lung sound as well, therefore, the present invention may be employed to monitor lung sound for breath sound analysis, respiratory health assessment. Similarity, the intestinal motility sound may also be monitored by the stethoscope when the device is attached to the intestinal tract. Apparently, the present invention provides motility frequency monitor, intensity analysis, and gastrointestinal functional status assessment.

The present invention provides multimode data integration, for example, the system simultaneously obtains the user's body temperature, heart sound signal (PCG), electrocardiogram signal (ECG), pulse wave signal (PPG), and blood oxygen saturation level (SpO₂). The user's body temperature is obtained by a thermometer 1306a (refer to FIG. 13(a), 13(b), 13(c), 13(d) and FIG. 14). An artificial intelligence (AI) prediction model is constructed through the fusion analysis of the multimode features (such as ECG R-R interval, heart sound S1-S2 interval, pulse wave rising time, and blood oxygen saturation changes).

The multimode data fetched by the present invention improves the accuracy and stability of blood pressure estimation, conducts cardiovascular health risk assessments (e.g., detect arrhythmias, early signs of valve disease), monitors respiratory health conditions (e.g. blood oxygen saturation and heart sound variation as indicators of respiratory abnormalities), analyze physiological stress load (e.g. estimating physical fatigue status from heart sounds and ECG variability).

Based on above features, the present invention provides novel heart sound analysis functions, for example, delay time analysis: the time interval between S1 and S2 could be used for preliminary detection of valvular disease and arrhythmia; heart sound variability analysis: statistical variation of time intervals between multiple heart sounds for pressure stress response analysis, trend change monitoring: long-term S1 and S2 change trends as cardiovascular health indicators.

In addition to the above advantages, the present invention also has the following technical features, which are superior to any existing technology, for example, in the device and application aspects: (1) the oximeter module of the present invention can operate independently. The oximeter module (including PPG sensing) can be an independent device with the functions of recording, storing and uploading heart rate and blood oxygen saturation data. The independent oximeter device can be attached to any part of the human body where PPG can be measured, including fingers, wrists, arms, etc. It has wireless transmission capabilities and may perform monitoring tasks independently without relying on the main device. (2) Telemedicine applications: the present invention can be applied to telemedicine-related applications, such as for home physiological monitoring of patients with chronic diseases (such as hypertension, cardiopulmonary diseases). Medical institutions can remotely receive heart sounds, blood pressure, SpO₂, HR and other data for health tracking, abnormal warning and remote intervention, highlighting the value of the present invention in telemedicine and long-term care applications.

In the signal processing and AI algorithm aspects: the present invention also provides multimode features, such as (1) Expansion of multimode for disease detection applications: the multimode (integration of PCG, ECG, PPG, SpO₂, etc.) can be applied to the detection and risk analysis of various diseases such as arrhythmia, heart failure, valvular disease, abnormal lung sounds, sleep apnea, etc., and improve the prediction accuracy of the model. (2) Benefits of multimode data in signal noise reduction processing: the present invention can perform dynamic filtering and noise reduction through cross-comparison of multimode signals (such as ECG and PCG timing, PPG and SpO₂ trends), thereby reducing noise caused by movement, poor contact or external interference, improving signal stability and data credibility, and enhancing the accuracy of subsequent analysis and diagnosis. The application of the multimode AI model of the present invention integrates PCG, ECG, PPG, SpO₂, etc. for disease detection and risk prediction. The algorithm and the architecture of the multimode AI model may be applied to improve the analysis capabilities for the multiple physiological states, such as arrhythmia, abnormal lung sounds, and sleep breathing disorders.

While various embodiments of the present invention have been described above, they have been presented by a way of example and not limitation. Numerous modifications and variations within the scope of the invention are possible. The present invention should only be defined in accordance with the following claims and their equivalents.

## Claims

1. A wearable physiological signal sensing system (1300) comprising:
a circuit board (1301) having an upper surface (1301b) and a lower surface (1301a);
a stethoscope (1302) formed on said lower surface (1301a) and electrically connected to said circuit board (1301) for sensing a user's (10) heart, lung or intestinal sound signal; and
a plurality of ECG electrodes (1304) disposed on said lower surface for sensing an ECG signal of said user.

2. The wearable physiological signal sensing system of claim 1, wherein an oximeter (1306) is arranged on said upper surface (1301b) for sensing a blood oxygen level and a pulse wave signal of said user (10).

3. The wearable physiological signal sensing system of claim 2, further comprising steps of comparing said ECG signal with said pulse wave signal, or comparing a heart sound signal with said pulse wave signal, for obtaining a pulse wave transit time of said user, and followed by calculating a continuous blood pressure of said user (10).

4. The wearable physiological signal sensing system of claim 3, wherein said continuous blood pressure is used to predict a blood pressure by an artificial intelligence (AI) algorithm.

5. The wearable physiological signal sensing system of claim 4, wherein a multivariate linear model is established by said artificial intelligence algorithm using a time domain characteristics of said pulse wave signal, said pulse wave transit time and a height of said user (10).

6. The wearable physiological signal sensing system of claim 2, wherein said oximeter (1306) includes an infrared light source, a red-light source and a photoreceptor for sensing a fingertip pulse wave of said user (10).

7. The wearable physiological signal sensing system of claim 6, wherein said user presses said oximeter (1306) to obtain said blood oxygen saturation level and said pulse wave signal.

8. The wearable physiological signal sensing system of claim 1, wherein said stethoscope (1302) includes a diaphragm (1302a) disposed on said lower surface (1301a) of said circuit board (1301).

9. The wearable physiological signal sensing system of claim 8, wherein a sound isolation ring (1302b) surrounds said diaphragm (1302a), thereby forming a resonance cavity with said circuit board (1301).

10. The wearable physiological signal sensing system of claim 1, wherein said wearable physiological signal sensing system (1300) is connected to an external mobile device (103), a cloud server (107) or an edge computing to transmit said physiological signal.

11. The wearable physiological signal sensing system of claim 10, further comprising an e-SIM formed on said circuit board (1301), wherein said wearable physiological signal sensing system (1300) is connected to an external mobile device (103), a cloud server (107) or an edge computing to transmit said physiological signal by said e-SIM.

12. The wearable physiological signal sensing system of claim 1, wherein said stethoscope (1302) collects biometrics identity.

13. The wearable physiological signal sensing system of claim 1, wherein said stethoscope (1302) performs heart rate analysis to determine whether it is an emergency.

14. The wearable physiological signal sensing system of claim 1, further comprising wireless charging coils (902).

15. The wearable physiological signal sensing system of claim 1, wherein said stethoscope (1302) includes a plurality of through holes (640) to allow sound to be received by a resonance cavity.
